# EUROPEAN PATENT APPLICATION

(11) **EP 2 966 468 A1**
(43) Date of publication of application: **13.01.2016**
(21) Application number: 15168824.9
(22) Date of filing: 22.12.2010
(51) Int. Cl.: G01S 7/52, G01S 15/89, G06T 17/00, G06T 19/00, A61B 8/08, A61B 8/14

(54) **PROVIDING AT LEAST ONE SLICE IMAGE BASED ON AT LEAST THREE POINTS IN AN ULTRASOUND SYSTEM**

(30) Priority: 12.01.2010 KR 20100002705
(62) Divisional of application: 10196496.3
(71) Applicant: Samsung Medison Co., Ltd., Hongcheon-gun, Gangwon-do (KR)
(72) Inventor: Kim, Sung Yoon, 250-870 Gangwon-do (KR)
(74) Representative: Schmid, Wolfgang

(57) **Abstract**

Embodiments for providing at least one slice image based on at least three points are disclosed. In one embodiment, by way of non-limiting example, an ultrasound system (100) comprises: an ultrasound data acquisition unit (110) configured to transmit and receive ultrasound signals to and from a target object to output ultrasound data; a user input unit (120) configured to receive input information for setting at least three points on a three-dimensional ultrasound image from a user; and a processing unit (130) in communication with the ultrasound data acquisition unit and the user input unit, the processing unit being configured to form volume data based on the ultrasound data, render the volume data to form the three-dimensional ultrasound image, set the at least three points on the three-dimensional ultrasound image based on the input information, set at least one slice on the three-dimensional ultrasound image based on the at least three points and form at least one slice image corresponding to the at least one slice.

## Description

### CROSS-REFERENCE TO RELATED APPLICATIONS

The present application claims priority from Korean Patent Application No. 10-2010-0002705 filed on January 12, 2010.

### TECHNICAL FIELD

The present disclosure generally relates to ultrasound systems, and more particularly to providing at least one slice image based on at least three points in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two-dimensional (2D) or three-dimensional (3D) ultrasound images of internal features of an object (e.g., human organs).

The ultrasound system may provide the 3D ultrasound image including clinical information such as spatial information and anatomical figures of the target object, which cannot be provided by the 2D ultrasound image. The ultrasound system may transmit ultrasound signals into a target object and receive ultrasound echo signals reflected from the target object. The ultrasound system may further form volume data based on the ultrasound echo signals. The ultrasound system may further render the volume data to thereby form the 3D ultrasound image.

However, to find a region of interest from the 3D ultrasound image, a user should rotate or move the 3D ultrasound image. Also, it may be difficult to provide at least one slice image including a plurality of regions of interest from the 3D ultrasound image.

### SUMMARY

Embodiments for providing a plurality of slice images in an ultrasound system are disclosed herein. In one embodiment, by way of non-limiting example, an ultrasound system comprises: an ultrasound data acquisition unit configured to transmit and receive ultrasound signals to and from a target object to output ultrasound data; a user input unit configured to receive input information for setting at least three points on a three-dimensional ultrasound image from a user; and a processing unit in communication with the ultrasound data acquisition unit and the user input unit, the processing unit being configured to form volume data based on the ultrasound data, render the volume data to form the three-dimensional ultrasound image, set the at least three points on the three-dimensional ultrasound image based on the input information, set at least one slice on the three-dimensional ultrasound image based on the at least three points and form at least one slice image corresponding to the at least one slice.

In another embodiment, there is provided a method of providing at least one slice image, comprising: a) transmitting and receiving ultrasound signals to and from a target object to output ultrasound data; b) forming volume data based on the ultrasound data; c) rendering the volume data to form the three-dimensional ultrasound image; d) receiving input information for setting at least three points on the three-dimensional ultrasound image from a user; e) setting the at least three points on the three-dimensional ultrasound image based on the input information; f) setting at least one slice on the three-dimensional ultrasound image based on the at least three points; and g) forming at least one slice image corresponding to the at least one slice.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a schematic diagram showing an example of acquiring ultrasound data corresponding to a plurality of frames.
FIG. 4 is a flow chart showing a process of forming at least one slice image based on at least three points set on a 3D ultrasound image.
FIG. 5 is a schematic diagram showing an example of volume data.
FIG. 6 is a schematic diagram showing an example of points set on the 3D ultrasound image.
FIG. 7 is a schematic diagram showing an example of slices set on the 3D ultrasound image.

### DETAILED DESCRIPTION

A detailed description may be provided with reference to the accompanying drawings.

One of ordinary skill in the art may realize that the following description is illustrative only and is not in any way limiting.

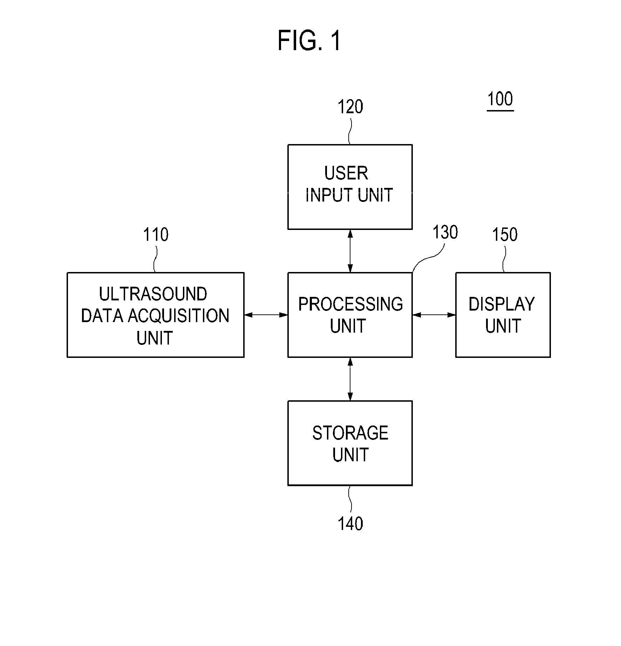

Referring to FIG. 1, an ultrasound system 100 in accordance with an illustrative embodiment is shown. As depicted therein, the ultrasound system 100 includes an ultrasound data acquisition unit 110. The ultrasound data acquisition unit 110 is configured to transmit and receive ultrasound signals to and from a target object to output ultrasound data.

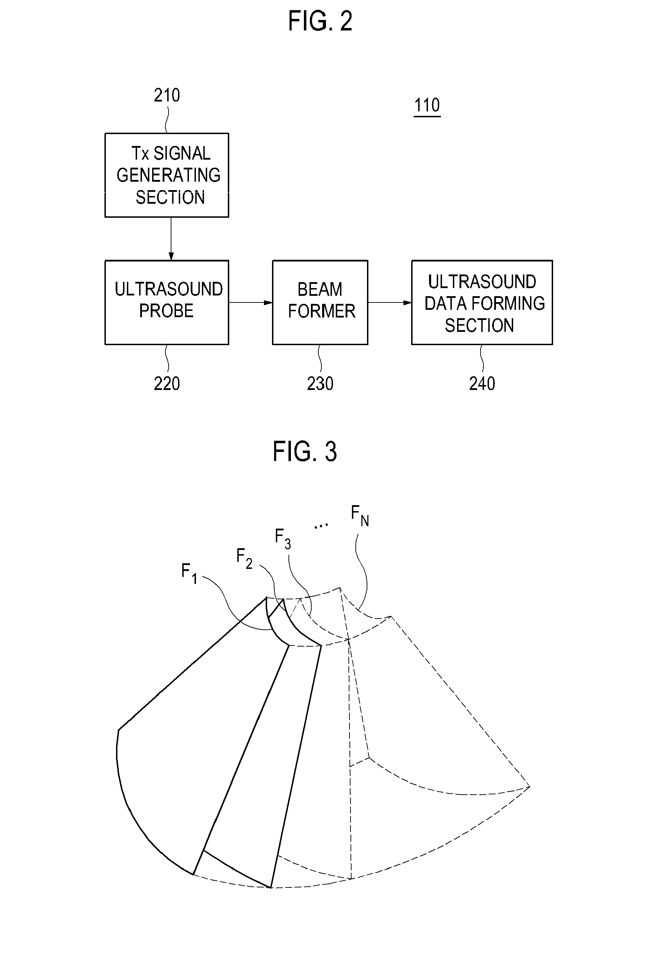

FIG. 2 is a block diagram showing an illustrative embodiment of the ultrasound data acquisition unit 110. Referring to FIG. 2, the ultrasound data acquisition unit 110 includes a transmit (Tx) signal generating section 210, an ultrasound probe 220, a beam former 230 and an ultrasound data forming section 240.

The Tx signal generating section 210 is configured to generate Tx signals. In one embodiment, the Tx signal generating section 210 generates Tx signals for obtaining a plurality of frames Fᵢ (1≤ i ≤ N) corresponding to a three-dimensional (3D) ultrasound image at every predetermined time, as shown in FIG. 3.

FIG. 3 is a schematic diagram showing an example of acquiring ultrasound data corresponding to the plurality of frames Fᵢ (1 ≤ i ≤ N). The plurality of frames Fᵢ (1 ≤ i ≤ N) represent sectional planes of the target object (not shown).

Referring back to FIG. 2, the ultrasound probe 220 includes a plurality of elements (not shown) for reciprocally converting between ultrasound signals and electrical signals. The ultrasound probe 220 is configured to transmit ultrasound signals to the target object in response to the Tx signals provided from the Tx signal generating section 210. The ultrasound probe 220 further receives ultrasound signals (i.e., ultrasound echo signals) from the target object to output received signals. The received signals are analog signals. The ultrasound probe 220 includes a three-dimensional (3D) mechanical probe or a two-dimensional (2D) array probe. However, it should be noted herein that the ultrasound probe 220 may not be limited thereto.

The beam former 230 is configured to convert the received signals provided from the ultrasound probe 220 into digital signals. The beam former 230 further applies delays to the digital signals in consideration of the elements and focal points to output digital receive-focused signals.

The ultrasound data forming section 240 is configured to form ultrasound data corresponding to the frames Fᵢ (1 ≤ i ≤ N) based on the digital receive-focused signals provided from the beam former 230. The ultrasound data forming section 240 further performs various signal processing (e.g., gain adjustment) upon the digital receive-focused signals.

Referring back to FIG. 1, the ultrasound system 100 further includes a user input unit 120. The user input unit 120 is configured to receive input information for setting at least three points on the 3D ultrasound image from a user. The user input unit 120 includes a control panel, a mouse or a keyboard. However, it should be noted herein that the user input unit 120 may not be limited thereto.

The ultrasound system 100 further includes a processing unit 130 in communication with the ultrasound data acquisition unit 110 and the user input unit 120. The processing unit 130 includes a central processing unit, a microprocessor or a graphic processing unit. However, it should be noted herein that the processing unit 130 may not be limited thereto.

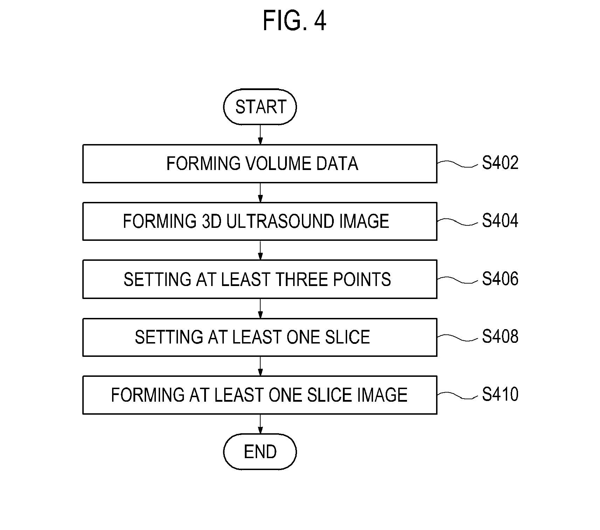

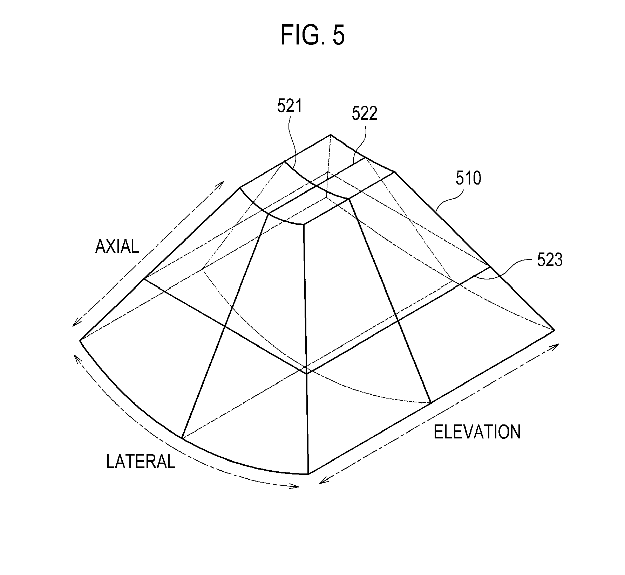

FIG. 4 is a flow chart showing a process of forming at least one slice image based on at least three points set on the 3D ultrasound image. The processing unit 130 is configured to synthesize the ultrasound data corresponding to the plurality of frames Fᵢ (1 ≤ i ≤ N) to form volume data 510 as shown in FIG. 5, at step S402 in FIG. 4.

FIG. 5 is a schematic diagram showing an example of the volume data 510. The volume data 510 includes a plurality of voxels (not shown) having brightness values. In FIG. 5, reference numerals 521, 522 and 523 represent an A plane, a B plane and a C plane, respectively. The A plane 521, the B plane 522 and the C plane 523 are mutually orthogonal. Also, in FIG. 5, the axial direction is a Tx direction of the ultrasound signals, the lateral direction is a longitudinal direction of the elements, and the elevation direction is a swing direction of the elements, i.e., a depth direction of the 3D ultrasound image.

The processing unit 130 is configured to render the volume data 510 to form the 3D ultrasound image, at step S404 in FIG. 4. The 3D ultrasound image is displayed on a display unit 150 in FIG. 1. Thus, the user sets the at least three points on the 3D ultrasound image displayed on the display unit 150 by using the user input unit 120.

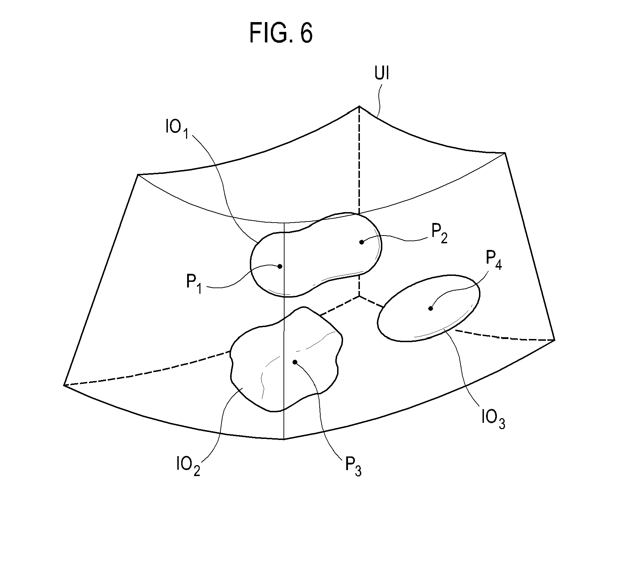

The processing unit 130 is configured to set the at least three points on the 3D ultrasound image based on the input information provided from the user input unit 120, at step S406 in FIG 4. In one embodiment, the processing unit 130 sets points P₁ to P₄ on corresponding positions of the 3D ultrasound image UI as shown in FIG. 6, based on the input information provided from the user input unit 120. In FIG. 6, reference numerals IO₁ to IO₃ represent objects of interest within the target object.

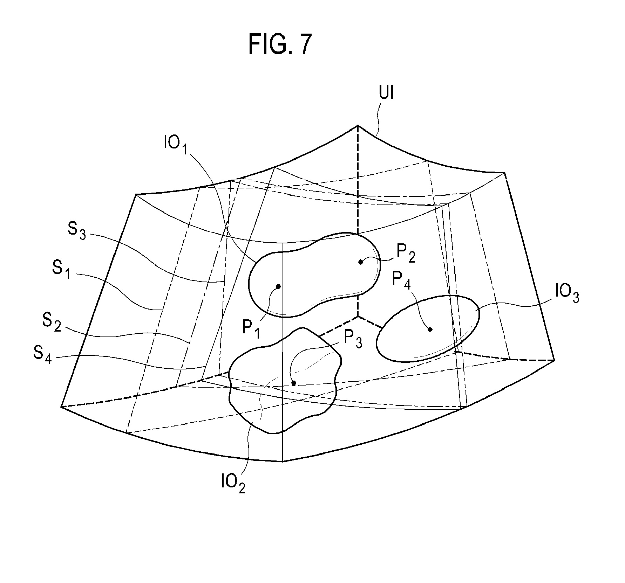

The processing unit 130 is configured to set at least one slice based on the at least three points set on the 3D ultrasound image, at step S408 in FIG. 4. In one embodiment, the processing unit 130 sets point groups from the points P₁ to P₄ that are set on the 3D ultrasound image UI as shown in FIG. 6, wherein each of the point groups includes three points. That is, the processing unit 130 sets a first point group including points P₁, P₂ and P₃ from the points P₁ to P₄, a second point group including points P₁, P₂ and P₄ from the points P₁ to P₄, a third point group including points P₁, P₃ and P₄ from the points P₁ to P₄, and a fourth point group including points P₂, P₃ and P₄ from the points P₁ to P₄. The processing unit 130 further sets a first slice S₁ passing through the points P₁, P₂ and P₃ of the first point group, a second slice S₂ passing through the points P₁, P₂ and P₄ of the second point group, a third slice S₃ passing through the points P₁, P₃ and P₄ of the third point group, and a fourth slice S₄ passing through the points P₂, P₃ and P₄ of the fourth point group, respectively, on the 3D ultrasound image UI as shown in FIG. 7.

The processing unit 130 is configured to form at least one slice image corresponding to the at least one slice, which is set on the 3D ultrasound image based on the volume data 510, at step S410 in FIG. 4. In one embodiment, the processing unit 130 is configured to form slice images corresponding to the slices S₁ to S₄ as shown in FIG. 7.

Referring back to Figure 1, the ultrasound system 100 further includes the storage unit 140. The storage unit 140 stores the ultrasound data acquired by the ultrasound data acquisition unit 110. The storage unit 140 further stores the volume data 510 formed by the processing unit 130.

The ultrasound system 100 further includes a display unit 150. The display unit 150 displays the 3D ultrasound image formed by the processing unit 130. The display unit 150 further displays the at least one slice image formed by the processing unit 130.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit and receive ultrasound signals to and from a target object to output ultrasound data;
a user input unit configured to receive input information for setting at least three points on a three-dimensional ultrasound image from a user; and
a processing unit in communication with the ultrasound data acquisition unit and the user input unit, the processing unit being configured to form volume data based on the ultrasound data, render the volume data to form the three-dimensional ultrasound image, set the at least three points on the three-dimensional ultrasound image based on the input information, set at least one slice on the three-dimensional ultrasound image based on the at least three points and form at least one slice image corresponding to the at least one slice.

2. The ultrasound system of Claim 1, wherein the processing unit is configured to:
set the at least one slice on the three-dimensional ultrasound image, wherein the at least one slice passes through three points, which are selected from the at least three points; and
form the at least one slice image corresponding to the at least one slice based on the volume data.

3. The ultrasound system of Claim 1, wherein the processing unit is configured to:
set at least one point group from the at least three points, wherein each of the at least one point group includes three points from the at least three points.

4. A method of providing at least one slice image, comprising:
a) transmitting and receiving ultrasound signals to and from a target object to output ultrasound data;
b) forming volume data based on the ultrasound data;
c) rendering the volume data to form the three-dimensional ultrasound image;
d) receiving input information for setting at least three points on the three-dimensional ultrasound image from a user;
e) setting the at least three points on the three-dimensional ultrasound image based on the input information;
f) setting at least one slice on the three-dimensional ultrasound image based on the at least three points; and
g) forming at least one slice image corresponding to the at least one slice.

5. The method of Claim 4, wherein the step f) comprises:
f1) setting the at least one slice on the three-dimensional ultrasound image, wherein the at least one slice passes through three points, which are selected from the at least three points; and
f2) forming the at least one slice image corresponding to the at least one slice based on the volume data.

6. The method of Claim 5, wherein the step f1) comprises:
setting at least one point group from the at least three points, wherein each of the at least one point group includes three points from the at least three points.

7. A computer readable medium comprising computer executable instructions configured to perform following acts:
a) forming volume data based on ultrasound data for a target object;
b) rendering the volume data to form the three-dimensional ultrasound image;
c) receiving input information for setting at least three points on the three-dimensional ultrasound image from a user;
d) setting the at least three points on the three-dimensional ultrasound image based on the input information;
e) setting at least one slice on the three-dimensional ultrasound image based on the at least three points; and
f) forming at least one slice image corresponding to the at least one slice.
